# EUROPEAN PATENT APPLICATION

(11) **EP 2 526 882 A1**
(43) Date of publication of application: **28.11.2012**
(21) Application number: 11167493.3
(22) Date of filing: 25.05.2011
(51) Int. Cl.: A61B 17/88

(54) **Surgical instrument for spreading bone**

(71) Applicant: VA-Vijf management BV, 8415 EK Deventer (NL)
(72) Inventor: Plugmacher, Robert, 12203, Berlin (DE); Becker, Gert, Waverley Pretoria (ZA); Van Asselt, Peter Gerrit Hendrikus, 7415 EK, Deventer (NL)
(74) Representative: De Vries & Metman

(57) **Abstract**

Surgical instrument (1) having a proximal portion and a distal portion, the distal portion arranged for separating tissue. The instrument comprises a substantially rigid first elongated member (3) and a second elongated member (5). The first and second members extend adjacent each other, and are longitudinally translatable along each other. In the distal portion, the first and second members are connected with a first lever (7), which is rotatable with respect to the first and second members about a first hinge (13) and a second hinge (15), respectively, such that by a longitudinal translation of the first and second members the lever rotates for spreading tissue and the angle (α) enclosed between the first member and an imaginary line crossing the first and second hinges increases.

## Description

### TECHNICAL FIELD

The present disclosure relates to medical instruments for spreading tissue, in particular surgical instruments for spreading bone, e.g. for treating compression fractures of the spine.

### BACKGROUND

Surgical instruments for spreading bone are known. For treatment of compression fractures of the spine a known technique comprises, using image guidance X-rays, that two small incisions are made and a probe is placed into the vertebral space where the fracture is located. The bone is drilled and a balloon, called a bone tamp, is inserted on each side. These balloons are then inflated with contrast medium (to be seen using image guidance x-rays) until they expand to the desired height and removed. The balloon does not remain in the patient. It simply creates a cavity for the cement and also helps expand the compressed bone.

The spaces created by the balloons are then filled with orthopaedic cement like PMMA, binding the fracture. The cement hardens quickly, providing strength and stability to the vertebra, restoring height, and relieving pain. The treatment is generally performed under local or general anaesthesia.

Instead of an inflatable balloon, which is expensive and may rupture, other devices for such treatment are known, e.g.: US 2008/0221608 provides a device comprising a housing having a lumen; a plunger having a proximal portion and a distal portion, where the plunger is disposed within the lumen and is movable relative to the housing; a plurality of blades, where the blades can expand radially from the axis of the housing; and a manipulator functionally connected to the plunger, wherein the manipulator is operable to: move the plunger relative to the housing; expand the blades radially from the axis of the housing; and move the blades about the axis of the plunger.

Variations to such device are e.g. known from WO 2008/137192 and US 2007/0173939.

Such devices are complex and delicate, rendering them expensive and hard to clean.

CN 2 868 219 Y relates to a clamp-type bone distractor for treating vertebral injury. It comprises a movable clamp handle, a fixed clamp handle, a clamp body and an upper clamp head. It further comprises a push rod fitted inside a recess of the lower part of the clamp body, which is arranged in a way that its rear end is pivotedly connected to the movable clamp handle via a hinge shaft and its front end pivotedly connected the upper clamp head via the hinge shaft and a connection rod.

The construction of the device is based on the well known surgical forceps and requires significant force for operation.

### SUMMARY

It is an object to provide an improved instrument of the aforementioned kind.

To that end, herewith a surgical instrument is provided, having a proximal portion and a distal portion, the distal portion comprising first and second contact portions arranged for separating tissue, in particular bony tissue e.g. vertebral bone. The instrument comprises a substantially rigid first elongated member and a second elongated member. The first and second members extend adjacent each other, in particular substantially parallel, and are longitudinally translatable along each other. In the distal portion, the first and second members are connected with a first lever, which is rotatable with respect to the first and second members about a first hinge and a second hinge, respectively, such that, by a longitudinal translation of the first and second members the lever rotates for spreading tissue and the angle (α) enclosed between the first member and an imaginary line crossing the first and second hinges increases.

The first member is coupled with, e.g. comprises the first contact portion. The first lever is coupled with, e.g. comprises the second contact portion, which may be comprised by the first lever, the second member and/or a further member. The hinging axes of the first and second hinges advantageously extend substantially parallel and perpendicular to the first member or at least the direction of the direction of translation of the first and second members.

Thus, upon a relative translation of the first and second members along the first member in the proximal portion the configuration of the instrument may be adapted and the tissue may be separated by the first lever in the distal portion. The first and second hinges act as a pivot and a contact point for exerting a torque on the first lever. The rotation (of the imaginary line crossing and indicating the relative positions of) the first and second hinges provides an increasing first lever arm on the first lever with increasing rotation of the first lever. Thus, at a constant translation force on the first and second members from the first to the second configuration, torque on the first lever increases with increasing rotation of the first lever and thus with increasing separation of the contact portions. Further, torque on the first lever by the tissue to be separated via the contact portion is reduces with increasing rotation of the first lever.

This facilitates use of the instrument and allows accurate control over the separation and the resulting separation force.

The instrument may be formed such that, in a first configuration the first lever extends substantially parallel to the first member and in a second configuration the first lever extends substantially perpendicular to the first member.

Such instrument allows a generally flat construction in the first configuration, facilitating insertion, whereas in the second configuration the tissue may be separated by a predetermined distance related to the length of the first lever and the tissue may be reliably supported in the predetermined distance without application of a translation force on the first and second members.

The first lever and the first and second hinges may be arranged, at least in a first configuration, on one side of the first member in particular the side towards the second contact portion.

Such instrument provides a single direction of separation and it facilitates determination of the position of the contact portions on the tissue.

The first lever may be at least partly received in the first and/or second member, preferably both. Preferably the first lever is, at least in a first configuration substantially fully enclosed by the first and second members. This allows reducing cross sectional size perpendicular to the first member and it allows increase "streamlining" the instrument, preventing (first lever) portions protruding from the instrument.

The second member may be deformable, e.g. being articulated and comprising at least a proximal section and a distal section movable to each other, e.g. hinged to each other. A chain, a flexible band or wire, e.g. of steel and/or a polymer, e.g. nylon or aramide may also be provided, suitable for exterting a pulling force on the first lever for rotating the first lever. In case of a band or a wire, the second hinge may comprise a band- or wire-loop attached to the first lever. When the second member comprises a proximal section and a substantially distal section movable to each other, preferably the distal section comprises the second hinge. Such instrument may be lighter weight than one comprising a substantially rigid second member. Further, it facilitates maintaining a constant, preferably small, separation between the first and second members in at least the proximal portion of the instrument, preventing accidental spreading of tissue.

The instrument may comprise a second lever hinged to the first lever, connected with, advantageously comprising, the second contact portion. Preferably the second lever is rotatably attached to the first lever remote from the first hinge. The second lever may be hinged to the first lever with the second hinge or with a separate third hinge. Thus, the centre of rotation of the second lever may be displaced with respect to the first member by the first lever. This provides increased freedom of manoeuvring and control of the second contact portion and it provides a further lever arm to the second contact portion between the second contact portion and the centre of rotation of the second lever, allowing to extend an effective lever arm between the second contact portion and the first hinge.

The second member may be articulated, comprising a proximal section and a distal section being rotatable to each other, wherein the distal section is hinged to the first lever and connected with the second contact portion, in particular comprising the second contact member. This provides a particularly efficient instrument which may comprise a relatively small number of parts and requires little volume. The instrument allows exertion of significant separation forces in a controlled manner. The instrument can have a positive relation between robustness and size.

To facilitate insertion, the distal portion, in at least one configuration, may comprise a substantially pointed or wedge-formed distal end.

For spreading force on the tissue, in at least one configuration the first and second contact portions may extend at a relatively small angle to each other, in particular substantially parallel to each other. E.g., the first member may comprise the first contact portion and the first lever and/or the second member may comprise the second contact portion. At least one of the first and second contact portions, in particular the second contact portion may comprise a bent portion, such that, in at least one configuration, the second contact portion extends at a relatively small angle to the first contact portion, e.g. substantially parallel. In case of rotatable first and/or second contact portions with respect to the first member such contact portion(s) may provide a relatively large contact surface at different separations.

The proximal portion of the instrument may be formed for manual operation of the distal end by providing a relative translation of the first and second members. E.g. the instrument may comprise a manual lever grip, a forceps or scissors-style ring grip, etc. with which the operator of the instrument will be familiar. This facilitates correct use in treatment.

In an embodiment, the proximal portion is provided with a connector for connecting one or more detachable operation devices, e.g. a detachable manual operation system, a spindle rotator, a motor, etc. possibly comprising a controller such as a computer controller. This facilitates exchange of instruments with continued use of the operation devices. It may further facilitate cleaning, maintenance and/or repair. It further allows combining the instrument with known apparel.

In the proximal portion of the instrument at least one the first and second members comprises a toothed and/or threaded section for adjusting the relative position of the first and second members by a rotary tool, e.g. a nut, crank or other handle rotatable about one of the first and second members connected to the other member for pulling or pushing the first and second members with respect to each other. This facilitates exerting a translation force on the first and second members with respect to each other in a controlled manner.

The first and second members of the instrument may be adjustable and (releasably) fixable in one or more configurations to maintain one or more predetermined separation distances.

Further, the proximal portion may comprise an indicator for indicating a separation distance provided by the distal portion, e.g. a ruler markings along the first and second members indicating their longitudinal translation, and/or a dial, which may be associated with adjustment via a rotary tool.

This increases control and or feedback on the operation of the device and progress of a treatment.

The instrument may comprise (releasably) paired ratchets or a ratchet and a (releasable) (snapping) pawl for setting and maintaining established increments without requiring external force on the instrument such as continued pinch gripping in case of a hand-operated instrument. Releasability facilitates retraction of the instrument.

A further aspect is a kit of parts comprising at least one disclosed instrument and bone cement, one or more bone cement precursors, and/or disposables. Such kits facilitate performing a treatment. Generally vertebral treatment is performed symmetrically so that the kit preferably comprises a pair of substantially identical instruments. Bone cement may be supplied as a multi-component product to be mixed shortly before or during treatment.

The kit may comprise a plurality of the instruments in different sizes, e.g. for different (stages of) ailments and/or (stages of) treatments. For treating compressed vertebrae the kit may comprise (a pair of) instruments with sizes of typically 4, 5 and /or 6 millimeter separation increase.

The instrument may be provided robust, and it may be reusable after sterilisation.

### BRIEF DESCRIPTION OF THE DRAWINGS

The above-described aspects will hereafter be more explained with further details and benefits with reference to the drawings showing an embodiment of the invention by way of example.
Figs. 1-6 show an instrument as herewith provided in different configurations;
Figs. 7-8 show an apparatus for operating the instrument in different configurations of;
Figs. 9 and 10 are side and perspective views of an embodiment in different configurations;
Figs. 11-19 show operation of the instrument of Figs. 9-10, with Figs. 12-14 and 17-19 being X-ray images of a successful prototype test.

### DETAILED DESCRIPTION OF EMBODIMENTS

It is noted that the drawings are schematic, not necessarily to scale and that details that are not required for understanding the present invention may have been omitted. The terms "upward", "downward", "below", "above", and the like relate to the embodiments as oriented in the drawings, unless otherwise specified. Further, elements that are at least substantially identical or that perform an at least substantially identical function are denoted by the same numeral.

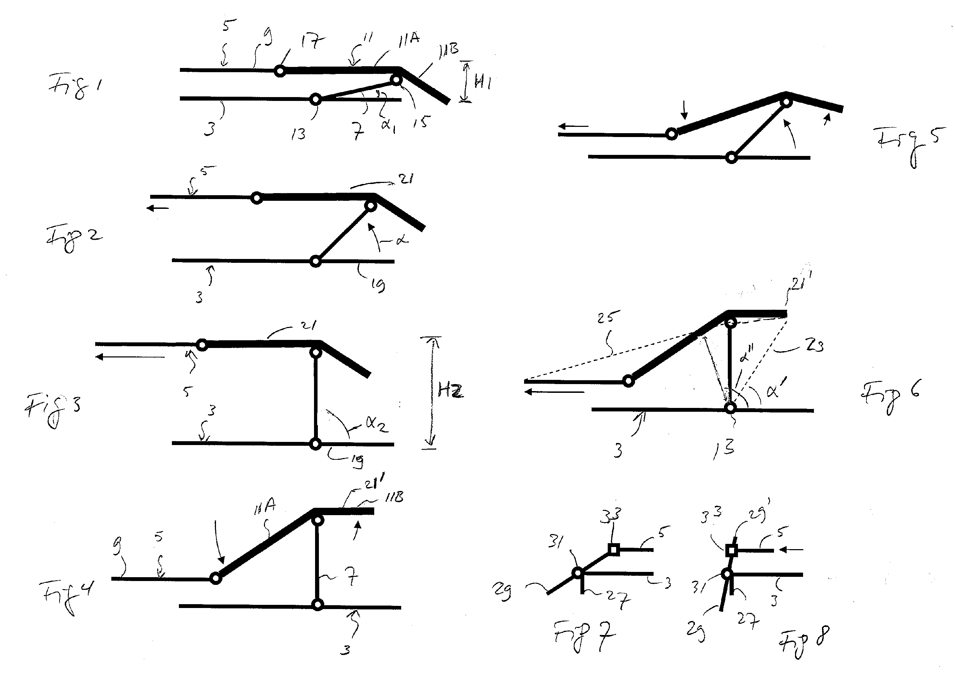

Figs. 1-6 indicate principles of operation of the instrument 1 with an exemplary design arranged in different configurations. The instrument 1 has a distal portion for separating tissue. All Figs. except for Figs 7-8 show the distal portion of the instrument 1. The instrument 1 further has a proximal portion for operating the instrument. Figs. 7-8 indicate a suitable design for the proximal portion.

The instrument 1 comprises a first elongated member 3, a second elongated member 5 and a first lever 7 arranged between the first and second members 3, 5. The first and second members 3, 5 extend adjacent and substantially parallel to each other. The second member is articulated, comprising a proximal section 9 and a distal section 11.

The first member 3 and the first lever 7 are rotatably connected with a first hinge 13. The first lever 7 and (the distal section 11 of) the second member 5 are rotatably connected with a second hinge 15. Thus the first lever 7 is rotatable with respect to the first and second members 3, 5 about the first and second hinges 13, 15, respectively.

The position of the second hinge 15 divides the distal section 11 of the second member 5 in a proximal leg 11A and a distal leg 11B. The proximal and distal sections 9, 11 of the second member 5 are rotatably connected with a third hinge 17.

The first, second and third hinges 13, 15, 17 all provide substantially parallel axes of rotation extending perpendicular to the plane of the Figures. Here, the first and second members 3, 5 and the first lever 7 are all substantially rigid.

Fig. 1 shows the distal portion of the instrument in a first configuration wherein the first and second members 3, 5 are arranged in a first relative longitudinal translational position and the first lever 7 extends in a small angle α1 to, and for a first, relatively small, distance from the first member 1. In this first configuration the distal portions of the first and second members 3, 5 determine a relatively small height H1.

Figs. 2-3 indicate with arrows that upon a relative translation of the first and second members 3, 5 the first lever 7 is erected on the first member 1 with an enclosed angle α and the first and second members 3, 5 are separated. The relative translation may be brought about by pushing against the first member 3 and/or pulling on the second member 5.

Fig. 3 shows the distal portion of the instrument in a second configuration wherein the first and second members 3, 5 are in a second relative translational position and the first lever extends in a second, relatively large angle 2 to, and for a second, relatively large, distance from the first member 1, here the first lever 7 extends substantially perpendicular to the first member (α2 is approximately 90 degrees). In this second configuration the distal portions of the first and second members 3, 5 determine a relatively large height H2.

Fig. 2 shows an intermediate situation between Figs. 1 and 3.

In the first configuration an imaginary line crossing the first and second hinges extends in a first, relatively small, angle (α1) with respect to the first member and in the second configuration the line extends in a second, relatively large angle (α2) with respect to the first member.

In use, the distal portions of the first and second members 3, 5 may be arranged between two portions of tissue to be separated by the instrument 1 in the first configuration of Fig. 1 and upon a translation of the first and second members 3, 5 to the first configuration of Fig. 3 the distal portions of the first and second members 3, 5 form contact portions 19, 21 contacting the tissue portions and the instrument can increase separation of the tissue portions for an extent D determined by the effective length L1 of the first lever 7 according to: D = L1.sin(α) (see the arrow D1 in Fig. 3).

The imaginary line crossing the first and second hinges 13, 15 determines a lever arm for acting on the first lever 7. In this instrument the lever arm Al on the first lever 7 provided by the first and second members 3, 5 is A1 = L1.sin(α) and is thus equal to the displacement. Hence application of a force F on the first lever by the by the first and second members 3, 5 produces a torque T = F.L1.sin(α) and the applied force thus acts substantially directly on the tissue to be separated allowing accurate control and direct feedback to the operator. In case the first lever 7 would be longer than the separation between the first and second hinges 13, 15, the displacement and the torque would have a corresponding constant ratio. The tissue counteracts the separating force with a lever arm A2 = L1.cos(α) and thus reduces with increasing separation.

Best seen in Fig. 4, an optional second lever is formed by (the proximal and distal legs 11A, 11B of) the distal section 11 of the second member 5, which here has a centre of rotation defined by the second hinge 15 and which is operable by translating the proximal section 9 of the second member 5 transversally relative to the first member 3. Depending on the shape (bent as shown here, bent over a larger or smaller angle or straight) and the relative lengths of the proximal and distal legs 11A, 11B, the second contact portion 21 may be shifted to a position marked 21' in Fig. 4. Also, the contact portions 19, 21' extend at a relatively small angle to each other, here being substantially parallel to each other.

Further, in the shown instrument, the effective height of the instrument and thus the tissue separation distance may be increased by rotating the second lever 11 beyond what is shown in Fig. 4. Fig. 5 indicates in dashed lines the resulting (virtual) first effective lever 23 with length L23 for separating tissue and a (virtual) second effective lever 25 for erecting the (virtual) effective lever 23 between the effective second contact point 21' and the first hinge 13. In Fig. 6, the first effective lever 23 is arranged at an angle α' with respect to the first hinge 13. The second effective lever 25 provides a lever arm 25" with length L25 arranged at an angle α" with respect to the first hinge 13.

Figs. 7 and 8 indicate an assembly for manual operation of the device, comprising a first and second gripping handles 27, 29. The first gripping handle 27 is fixed to the first member 3, the second gripping handle 29 is hinged to the gripping handle 27 with a fixed hinge 31 and is hinged to the second member 5 with a slidable hinge 33. Upon squeezing the handles together the second member is pulled with respect to the first member 3, 5 and the configuration of the assembly is changed (Fig. 7 to Fig. 8) providing a relative translation between the first and second members 3, 5 for operating the distal portion is provided. The slidable hinge 33 prevents a transverse separation of the first and second members 3, 5 upon operation, see the portion 29' extending from the slidable hinge 33. Instead of being directly coupled to the first and second members 3, 5, the assembly may comprise a connector to connect releasably to the first and second members 3, 5, or individual connectors for each one of the first and second members 3, 5 (neither shown). Other types of levers may be provided as well.

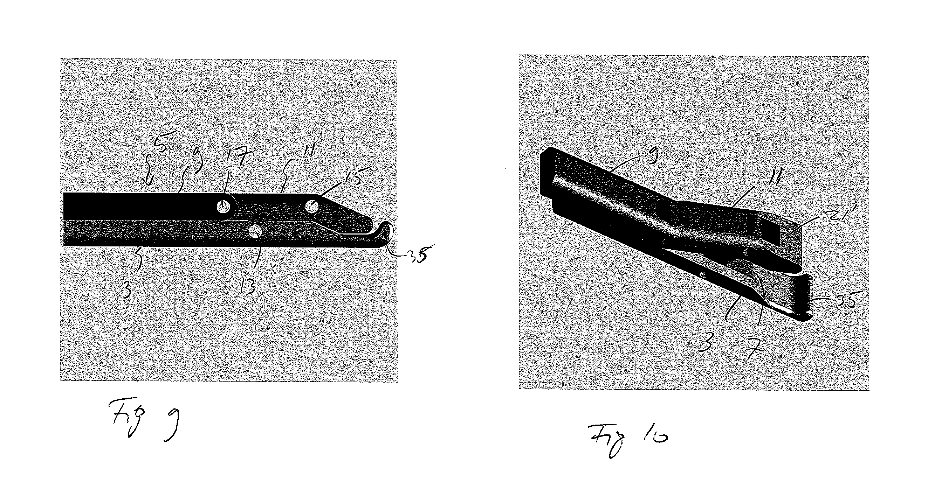

Figs. 9-10 show the distal portion of an embodiment of the instrument 1, which encloses the lever 7 in the first, closed, configuration (Fig. 9). The instrument has a generally wedge-shaped distal tip. Here, the first member 3 comprises a nose 35 protruding distally beyond the second member 5. The instrument is substantially rectangular with smoothly rounded corners in cross section perpendicular to the direction of extension. This allows a secure placement, provides a large contact surface and/or prevents it from rotating or toppling. The instrument is radiopaque for X-rays, e.g. metallic or high density polymeric, providing good visibility in X-ray images. It also is robust against interaction with the patients body and/or aging.

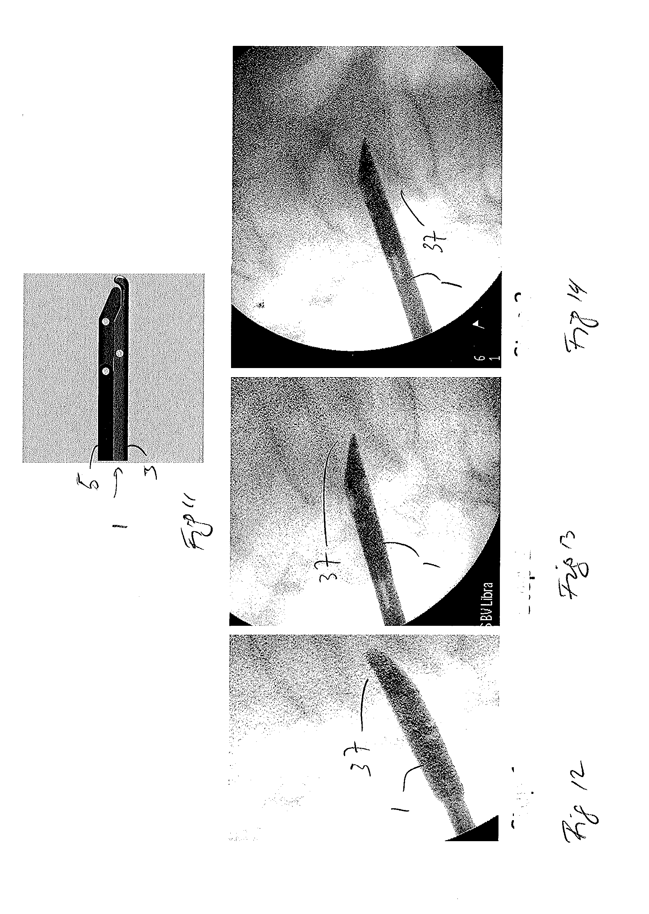

Fig. 11 is generally identical to Fig. 9 and is shown for reference purposes. Figs. 12-14 are X-ray images showing insertion of the instrument into a pre-drilled cavity in a vertebral body 37 suffering from compression fracture. The wedge-shaped tip facilitates the insertion into the body both in the bone and in the soft tissue. It further allows providing a space for the instrument by hammering the instrument into the bone. The articulated second member 5 is protected against the hammering blows into the bone by the nose 35 of the first member.

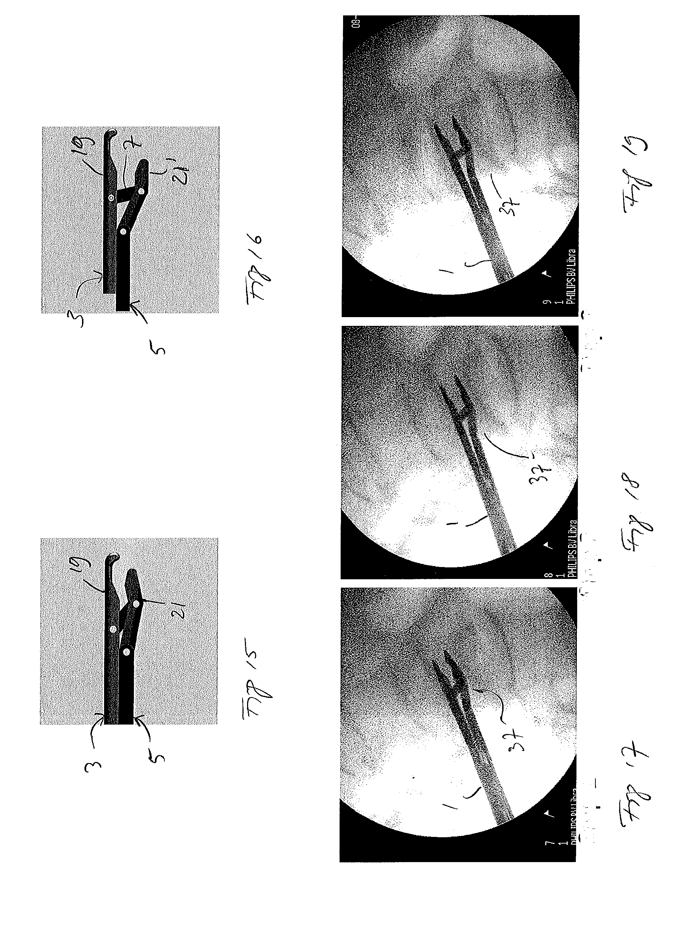

Upon proper placement of the instrument, the first and second members are translated along each other and the distalportion is opened as described above and as shown in Figs. 15-19. Note that in these Figs. the instrument 1 is inverted relative to the images before. The superior and inferior portions of the hard bony shell of the treated vertebral body are pressed outward by the contact portions 19, 21, 21' of the instrument. Due to the treatment, and a cavity is formed in the vertebral body. If need be, the cavity may be expanded by repeating the operation with a further substantially identical instrument but of a larger size, in particular comprising a first lever with a larger length. A subsequently used instrument may not comprise a nose 35. The (expanded) cavity may thereafter be filled with bone cement in known manner.

The invention is not restricted to the above described embodiments which can be varied in a number of ways within the scope of the claims. For instance, the instrument may comprise further contact portions and or further levers, similarly arranged with respect to the first member and which possibly may extend from the first member in different, possibly opposite, directions, which may facilitate providing a void in the tissue. Two levers extending an operating in opposite direction facilitates providing the same transverse displacement at less, typically half, the longitudinal displacement of an instrument having a single lever or at least a lever extending to a single side from the first member as shown in the Figures.

In case of an articulated second member, the distal section need not extend distally beyond the first lever and/or second hinge. This provides the benefit of a narrow proximal section even lacking the second lever functionality.

In case of an articulated second member, the first and second members may comprise a guide, e.g. comprising a ring, a clip or interlocking portions, for maintaining constant lateral distance between the first member and the proximal portion of the second member.

The instrument may serve also for separating other tissue portions, e.g. for spreading ribs and/or setting bone fractures.

Elements and aspects discussed for or in relation with a particular embodiment may be suitably combined with elements and aspects of other embodiments, unless explicitly stated otherwise.

## Claims

1. Surgical instrument (1) having a proximal portion and a distal portion, the distal portion comprising a first and a second contact portion (19, 21, 21') arranged for separating tissue,
the instrument comprising a substantially rigid first elongated member (3) and a second elongated member (5),
wherein the first and second members extend adjacent each other and are longitudinally translatable along each other,
wherein in the distal portion, the first and second members are connected with a first lever (7), which is rotatable with respect to the first and second members about a first hinge (13) and a second hinge (15), respectively, such that, by a longitudinal translation of the first and second members the lever rotates for spreading tissue and the angle (α) enclosed between the first member and an imaginary line crossing the first and second hinges increases.

2. Instrument (1) according to claim 1, wherein in a first configuration the first lever (7) extends substantially parallel to the first member (3) and in a second configuration the first lever extends substantially perpendicular to the first member.

3. Instrument (1) according to any preceding claim, wherein at least in a first configuration the first lever (7) and the first and second hinges (13, 15) are arranged on one side of the first member.

4. Instrument (1) according to any preceding claim, wherein the first lever (7) is at least partly received in the first and/or second member (3, 5), preferably the first lever is, at least in one configuration, enclosed by the first and second members.

5. Instrument (1) according to any preceding claim, wherein the second member (5) is deformable.

6. Instrument (1) according to any preceding claim, comprising a second lever (11) hinged to the first lever (7) and connected with the second contact portion (21, 21').

7. Instrument (1) according to claims 5 and 6, wherein the first member comprises a first contact portion (19) and the second member (5) is articulated, comprising a proximal section (9) and a distal section (11) being rotatable to each other, wherein the distal section is hinged to the first lever (7) and connected with a second contact portion (21, 21').

8. Instrument (1) according to any preceding claim, wherein the distal portion, in at least one configuration, comprises a substantially pointed or wedge-formed distal end.

9. Instrument (1) according to any preceding claim, wherein in at least one configuration the first and second contact portions (19, 21, 21') extend at a relatively small angle to each other, in particular substantially parallel to each other.

10. Instrument (1) according to any preceding claim, wherein the proximal portion is formed for manual operation of the distal end by providing a relative translation of the first and second members (3, 5).

11. Instrument (1) according to any preceding claim, wherein the proximal portion is provided with a connector for connecting one or more detachable operation devices.

12. Instrument (1) according to any preceding claim, wherein in the proximal portion of at least one the first and second members (3, 5) comprises a toothed and/or threaded section for adjusting the relative position of the first and second members by a rotary tool.

13. Instrument (1) according to any preceding claim, being adjustable and fixable in one or more configurations to maintain one or more predetermined separation distances and/or wherein the proximal portion comprises an indicator for indicating a separation distance provided by the distal portion.

14. Kit of parts comprising at least one instrument (1) of any preceding claim and bone cement, one or more bone cement precursors, and/or disposables.

15. Kit according to claim 14 comprising a plurality of instruments (1) of any one of claims 1-12 in different sizes.
